# EUROPEAN PATENT SPECIFICATION

(11) **EP 2 986 732 B1**
(45) Date of publication and mention of the grant of the patent: **10.10.2018**
(21) Application number: 14784907.9
(22) Date of filing: 18.04.2014
(51) Int. Cl.: C12Q 1/00, B01J 13/18

(54) **LASER MARKING FOR AUTHENTICATION AND TRACKING**
LASERMARKIERUNG ZUR AUTHENTIFIZIERUNG UND VERFOLGUNG
MARQUAGE LASER POUR AUTHENTIFICATION ET SUIVI

(30) Priority: 18.04.2013 US 201361813578 P
(43) Date of publication of application: 24.02.2016
(73) Proprietor: Applied DNA Sciences Inc., Stony Brook, NY 11790 (US)
(72) Inventor: TRAN, Phidung, H., East Setauket, NY 11733 (US); JUNG, Lawrence, Forest Hills, NY 11375 (US); LIANG, Benjamin, MingHwa, East Setauket, NY 11733 (US); HAYWARD, James, A., Stony Brook, NY 11790 (US)
(74) Representative: HGF Limited
(86) International application number: PCT/US2014/034642
(87) International publication number: WO 2014/172630

(56) References cited:
- US-A- 4 861 620
- US-A1- 2002 185 634
- US-A1- 2008 299 559
- US-A1- 2009 042 191
- CHRISEY L A ET AL: "FABRICATION OF PATTERNED DNA SURFACES", NUCLEIC ACIDS RESEARCH, INFORMATION RETRIEVAL LTD, GB, vol. 24, no. 15, 1 January 1996 (1996-01-01), pages 3040-3047, XP002913337, ISSN: 0305-1048, DOI: 10.1093/NAR/24.15.3040
- KHANDJIAN E W: "OPTIMIZED HYBRIDIZATION OF DNA BLOTTED AND FIXED TO NITROCELLULOSE AND NYLON MEMBRANES", BIO-TECHNOLOGY (NEW YORK), vol. 5, no. 2, 1987, pages 165-167, XP002762699, ISSN: 0733-222X
- ALEKSANDR OVSIANIKOV ET AL: "Two-photon polymerization technique for microfabrication of CAD-designed 3D scaffolds from commercially available photosensitive materials", JOURNAL OF TISSUE ENGINEERING AND REGENERATIVE MEDICINE, vol. 1, no. 6, 1 November 2007 (2007-11-01), pages 443-449, XP055308966, US ISSN: 1932-6254, DOI: 10.1002/term.57
- Jim Hayward ET AL: "A Scaled, Integrative Implementation for DNA Marking of Integrated Circuits", , 18 April 2013 (2013-04-18), XP055308145, Retrieved from the Internet: URL:http://www.erai.com/CustomUploads/conf erence/2013/PDF/APDNAApril18.pdf
- F FIXE ET AL: "Thin film micro arrays with immobilized DNA for hybridization analysis", MRS ONLINE PROCEEDINGS, vol. 723, 1 January 2002 (2002-01-01), pages O2.3.1-O2.3.6, XP055308987, US ISSN: 1946-4274, DOI: 10.1557/PROC-723-O2.3
- ANDRE G. SKIRTACH ET AL.: 'The Role of Metal Nanoparticles in Remote Release of Encapsulated Materials''.' NANO LETT. vol. 5, no. 7, 2005, pages 1371 - 1377, XP055226854
- FRANCK THIBAUDAU: 'Ultrafast Photothermal Release of DNA from Gold Nanoparticles''.' J. PHYS. CHEM. LETT. vol. 3, 2012, pages 902 - 907, XP055286693

## Description

### FIELD OF THE INVENTION

This invention relates to the incorporation of security markers, such as DNA, and/or other biomolecules on the surface of or within the matrix of a substrate using LASER marking and the use of such marked surfaces for authentication and tracking of valuable objects and other items of interest.

### BACKGROUND

LASER (Light Amplification by Stimulated Emission of Radiation) marking can be used to mark a variety of substrates. For instance, several different methods for laser markings are disclosed in U.S. Patent No. 4,861,620 to Azuma. When the LASER interacts with a surface, the surface is transformed by melting, vaporization, or by chemical reaction that can be used to convert coated dye material on or encapsulated inside the substrate in an engraving mark, and provide a contrasting mark or color change from the surrounding.

LASERs have been used for marking of molding compounds such as electronic devices, see for instance, U.S. Patent No 4,654,290 to Spanjer. These processes provide security markings using a LASER for printing small covert marks that are readily discernible at high magnification.

US 4,861,620 discloses a method of laser marking objects.

### SUMMARY OF THE INVENTION

Here we disclose several different methods for incorporation and/or immobilization of security markers by exposure to an electromagnetic pulse, to produce a marked object that can be authenticated only by using proprietary biological, chemical or physical analysis.

In one aspect the disclosure relates to a method of marking an object, the method includes: exposing a surface of the object to be marked to an electromagnetic pulse, such as for instance a LASER pulse, to activate the surface, and exposing the activated surface to a detectable marker molecule and thereby immobilizing the detectable marker molecule on the surface of the object. In one embodiment, the surface of the object is partially melted by the electromagnetic pulse.

The invention provides a method of marking an object, wherein the method includes: providing an object wherein at least a portion of the surface is coated with a detectable marker molecule; and exposing a surface of the object to a LASER pulse, sufficient to immobilize the detectable marker molecule on the surface of the object.

In another aspect the disclosure relates to a method of marking an object, including exposing a surface of the object to be marked to a LASER pulse to chemically activate the surface, and exposing the activated surface to a detectable marker molecule and thereby immobilizing the detectable marker molecule on the surface of the object.

In another aspect the disclosure relates a method of marking an object having a coating including a photo-polymerizable monomer and a detectable marker molecule over at least a portion of its surface; wherein the method includes exposing the coated portion of the surface of the object to be marked with a LASER pulse to polymerize the coating, and thereby immobilizing the detectable marker molecule on the surface of the object.

Further disclosed is an object marked with a detectable marker, produced by a method that includes: providing an object, wherein at least a portion of the surface is coated with a detectable marker molecule; exposing the coated surface of the object to an electromagnetic pulse sufficient to activate the surface, immobilizing the detectable marker molecule on the surface of the object; thereby producing an object marked on at least a portion of its surface with a detectable marker molecule.

### DETAILED DESCRIPTION

In one aspect the disclosure relates to a method of marking an object, including exposing a surface of the object to be marked to a LASER pulse to activate the surface, and exposing the activated surface to a detectable marker molecule and thereby immobilizing the detectable marker molecule on the surface of the object, wherein the detectable marker molecule includes one or more of a biomolecule, a dye, a fluorophore, a metal a trace element and a rare earth element. The biomolecule can be any suitable biomolecule, such as for instance and without limitation, one or more of a nucleic acid, a protein, a peptide, a co-enzyme and a vitamin. In a particular embodiment, the detectable marker biomolecule includes DNA. The biomolecule can be any suitable biomolecule, such as for instance a biomolecule from an animal, a plant, a fungus, a bacterium a virus or other biological organism.

In another aspect the biomolecule is immobilized in a carrier medium such as a cyanoacrylate medium on a surface. In one embodiment the biomolecule is present in the carrier medium in a range of from about 0.1 ppm to about 10,000 ppm by weight.

In another aspect the disclosure relates to a method of marking an object, including exposing a surface of the object to be marked to an electromagnetic pulse to activate the surface, and exposing the activated surface to a detectable marker molecule and thereby immobilizing the detectable marker molecule on the surface of the object, wherein the electromagnetic pulse produces activated functional groups on the surface of the object. In an alternative aspect, the electromagnetic pulse produces a plasma at the surface of the object.

In still aspect the disclosure relates to a method of marking an object, including exposing a surface of the object to be marked to an electromagnetic pulse to activate the surface, and exposing the activated surface to a detectable marker molecule and thereby immobilizing the detectable marker molecule on the surface of the object, wherein the detectable marker molecule is immobilized on the surface of the object by chemical bonding.

In another aspect the disclosure relates to a method of marking an object, including exposing a surface of the object to be marked to an electromagnetic pulse to activate the surface, and exposing the activated surface to a detectable marker molecule and thereby immobilizing the detectable marker molecule on the surface of the object, wherein the detectable marker molecule is provided in or on a medium that is melted onto the surface of the object by the electromagnetic pulse.

The medium in or on which the detectable marker molecule is provided can be any suitable form of the medium, such as for instance and without limitation, the detectable marker molecule can include one or more of a film, a thread, a capsule, a bead and an appliqué, such as a shaped appliqué in the form of a design, such as a trademark or other recognizable design. The medium in or on which the detectable marker molecule is provided can be of any suitable medium, such as for instance and without limitation, the medium in or on which the detectable marker molecule is provided can include any suitable resin or polymer, such as one or more of a plastic, a cyanoacrylate and an epoxy polymer. Alternatively, the medium in which the detectable marker molecule is provided can be an aqueous solution.

In one embodiment, the invention provides a method of marking an object having a coating including a photo-polymerizable monomer and a detectable marker molecule over at least a portion of its surface; wherein the monomer is transparent to all but very high intensity light, but is activated by a very high intensity electromagnetic pulse such as a very short pulse, e.g. one fempto-second, i.e. 10⁻¹⁵ seconds of a high intensity LASER with a power of the order of from 1-200 GW (GigaWatts: 10⁹ Watts) sufficient for two photons to interact with a single photo-polymerizable monomer molecule. Such LASER pulses initiate polymerization in a two-photon mediated polymerization reaction mediated by short-lived free radicals formed by the LASER pulse. The LASER useful in the practice of the present invention can be any suitable LASER, such as for instance, an infrared (IR) LASER, an ultraviolet (UV) LASER, or a visible LASER.

The phenomenon of two-photon absorption by a single molecule was first predicted in the 1930's and first observed in the 1960's after the introduction of pulsed LASERs with sufficient power to provide two photons in the very narrow window of time and space to interact with a single molecule. It has only recently become routinely possible to reach sufficiently high peak intensities of the order of 10-100 GW (corresponding to 1-10% of the peak electrical power capacity for the entire United States) for very short periods of time of the order of fempto seconds (i.e one millionth of a nanosecond) to initiate a two-photon absorption and subsequent chemical reaction. The two-photon absorption can lead to radical formation, particularly in molecules having electron donor or electron withdrawing groups. Since radicals are reactive species, they can link otherwise stable and unreactive monomers to form polymers.

The two-photon absorption of light by a molecule is inversely dependent on the square of the light intensity, in contrast to one-photon absorption which bears a linear relationship to the inverse of the light intensity. This results in a rapid fall off over nanometer scale distances of the effective two-photon absorption and hence the focusing of polymerization reactions initiated by the high intensity LASER beam. Such sharp focusing of the polymerization initiation region in a volume pixel (voxel) with dimensions of the order of 100 nanometers (nm) permits ultra-fine control of the solid formed by polymerization of the monomer solution or gel useful for two dimensional (2D) high resolution printing and three dimensional (3D) fabrication. Two-photon polymerization reactions can be used for tracking, authentication or validation of valuable items, commodities in commerce or items in transit for instance by micro-scale printing of or embedding of security labels or tags incorporating trapped or encapsulated detectable markers. Such detectable markers can be trapped in a polymer molecular network or in a specifically polymerized 3D mesh such as a honeycomb or other 3D structure having cells encapsulating the detectable marker.

Liquids or gels that contain a photo-polymerizable monomer that is transparent to all but very high intensity light, can be activated by a focused nanoscale finely controlled very high intensity LASER or other electromagnetic pulse, initiating polymerization according to the path of the pulse in the liquid or gel to produce any shape or object form. This is the basis of many three dimensional (3D) printers. Photoinitiators, molecules with a low photo-dissociation energy can be added to increase the photosensitivity of the polymerizable material. There are three major classes of photo-initiators determined by the molecular cleavage mechanism which depends on radical formation in the photoinitiator occuring by photocleavage, hydrogen abstraction or cationic polymerization.

Two-photon absorption initiates polymerization of monomers having unsaturated bonds, especially delocalized (i.e. π-conjugated) double bonds, such as vinyl and styrene derivatives. Characteristics of the polymer can be tailored by choice of substituents (electron donating or accepting groups according to the ultimate use) of the conjugated system, to provide any desired degree of solubility, lipophilicity and absorption properties of the monomer. Examples of suitable monomers include for instance and without limitation, methacrlyic and acrylic derivatives of polyethylene glycol (PEG), ethylene glycol-lactic acid copolymers, urethanes, and poly(anhydrides).

In one aspect the disclosure relates to a method of marking an object, including exposing a surface of the object to be marked to an electromagnetic pulse to activate the surface, and exposing the activated surface to a detectable marker molecule and thereby immobilizing the detectable marker molecule on the surface of the object, wherein the detectable marker molecule can be detected by any suitable detection method, such as one or more of visible light, infrared light, UV light, fluorescence and phosphorescence. For instance molecules with two or more electron donors such as amino or alkoxy substituents of aromatic or hetero-aromatic groups as part of a π-conjugated bond system are even more effective as two-photon absorption molecules than the commonly used dyes such as styryl dyes, stilbene derivatives, diphenyl polyenes, phenylene vinylene oligomers, rhodamine and related molecules. Conjugation of electron accepting groups such as cyano, formyl or dicyanomethylidene can also enhance the two-photon absorptivity.

In another aspect the disclosure relates to a method of marking an object, including exposing a surface of the object to be marked to an electromagnetic pulse to activate the surface, and exposing the activated surface to a detectable marker molecule and thereby immobilizing the detectable marker molecule on the surface of the object, wherein the detectable marker molecule includes DNA and wherein the DNA is detected by hybridization, amplification or nucleotide sequencing. The DNA can be any suitable DNA, such as for instance and without limitation, the DNA can be a natural or a non-natural single-stranded DNA molecule or a double-stranded DNA molecule having a sequence of from 10 to 10,000 nucleotides. In another aspect the DNA can be a natural or a non-natural, single-stranded DNA or double-stranded DNA of 15 to 500 nucleotides. In yet another embodiment the DNA can include a natural or a non-natural sequence of 20 to 250 nucleotides. In a particular embodiment the detectable marker molecule includes a DNA molecule and a visible marker and/or an optical reporter. In one embodiment, the marker DNA molecule is included with an excess of carrier DNA of a different composition or sequence to "hide" or camouflage the marker DNA. For instance the carrier DNA may be in one hundred fold, one thousand fold, ten thousand fold, one hundred thousand fold, or one million fold or more in excess by weight over the weight of marker DNA.

Also disclosed is an object that includes an immobilized detectable marker molecule, the object having been produced by the methods of the present invention. In one aspect, the detectable marker molecule immobilized on the object is immobilized on an activated surface of the object, the activated surface having been produced by exposure to an electromagnetic pulse.

In another aspect, the immobilized detectable marker molecule includes one or more of a biomolecule, a dye, a fluorophore, a metal, a trace element and a rare earth element. The biomolecule can be any suitable biomolecule, such as for instance and without limitation, one or more of a nucleic acid, a protein, a peptide, a carbohydrate, a co-enzyme and a vitamin. In a particular embodiment, the nucleic acid detectable marker biomolecule includes DNA.

The DNA markers of the present disclosure can encode or be used to correspond to manufacturer information such as for instance and without limitation, a unique serial number of the item, the make and model of the item as well as such detail as the date of manufacture or date of shipping and the identification and provenance of components used in its manufacture. Each component sequence or subsequence of the DNA marker can be used to denote a different item of information relevant to the item or its components. DNA markers can also provide authentication and tracking at any point in the supply chain and in the stream of commerce. In another alternative, new DNA markers can be added by affixing or printing with marker DNA encoding new data during manufacture or in the stream of commerce for maintenance of a continuous record of chain of custody of the item.

DNA markers, such as botanical-DNA based markers for security and authentication uses can help protect products, brands and intellectual property of companies, governments and consumers from theft, counterfeiting, fraud and diversion. These DNA markers have an almost unlimited coding capacity which essentially cannot be reverse engineered, and which provides forensic evidence that can be used in the prosecution of thieves, counterfeiters and perpetrators of fraud and diversion.

DNA marking for security and authentication is readily applied to mass produced items such as microelectronic components due to the ease with which the DNA marker can be applied by a wide variety of immobilization methods of the present invention using manual, automated or semi-automated equipment. In one alternative, the DNA marker or markers, such as botanical-DNA based markers can be affixed to packaging, such as tamper-proof packaging in addition to or instead of marking the packaged item itself.

DNA markers suitable for use in the methods of the present invention can be prepared as described in U.S. Patent 8,426,216 to Kwok. Briefly, in certain embodiments, the DNA marker is derived from DNA extracted from a specific plant source and is specifically digested and the fragments produced are then ligated in random order to generate artificial nucleic acid sequences which are unique to the world. The digestion and ligation of the extracted DNA is completed by standard restriction digestion and ligase techniques known to those skilled in the art of molecular biology. An optical reporter marker deposited on the item along with the DNA marker also enables the authentication of the article of interest by both confirming that the correct emission spectra/wavelength for the optical reporter is detected as well as facilitating the location of the DNA marker, enabling sequencing if the DNA marker includes the correct nucleic acid sequence. The optical reporter marker may camouflage or "hide" a specified DNA marker of verifiable sequence by including extraneous and nonspecific nucleic acid oligomers/fragments, thus making it difficult for unauthorized individuals such as forgers to identify the sequence of the DNA marker. The optical reporter marker can include a specified double-stranded DNA marker from a known source (such as a mammal, invertebrate, plant or the like) along with genomic DNA from the corresponding or similar DNA source. The amount of the DNA marker incorporated in or on an object of interest with an optical reporter marker compound may vary depending on the article to be authenticated, the duration or shelf-life the taggant needs to be viable (e.g. 1 day, 1 month, 1 year, multiple years) prior to authentication, expected environmental exposure, the detection method to be utilized, and other factors.

Other reporters useful in the practice of the present invention include chemical reporters, such as small molecule markers that can be identified with well known and widely available basic chemistry. In an alternative embodiment, optical marker dye(s), fluorescent or phosphorescent marker compounds can be used and can be detected visually, with ultraviolet light or in the dark after light exposure, respectively.

In one embodiment, the DNA sequence of the marker DNA is encoded in an encrypted digital code such as for instance a bar code, a radio-frequency ID code (RFID), a quick read (QR) code or other visually readable or instrument-readable code.

Unique DNA markers may be synthetically produced using a nucleic acid synthesizer. Alternatively, DNA can be isolated from any organism such as yeast, human cell lines, bacteria, animals and plants. In certain embodiments, the nucleic acid material may be treated with restriction enzymes and then purified to produce an acceptable nucleic acid marker(s). The length of the nucleic acid marker/tag usually ranges between about 100 to about 10 kilo bases, more usually about 500 bases to about 6 kb, or about 1 kb to about 3 kb in length.

The DNA markers may comprise one specific nucleic acid sequence or alternatively, may comprise a plurality of various nucleic acid sequences. In one embodiment, polymorphic DNA fragments of the type short tandem repeats (STR) or single nucleotide polymorphisms (SNP) are utilized as an anti-counterfeit DNA marker. While the use of a single sequence for a nucleic acid marker may make detection of the marker easier and quicker, the use of a plurality of nucleic acid sequences such as STR and SNP, in general, give a higher degree of security against forgers.

The nucleic acid (NA) marker useful as a taggant can be DNA, cDNA, or any other nucleic acid fragment comprising nucleic acids or nucleic acid derivatives. The NA maybe a nucleic acid fragment that is single stranded or preferably double stranded and may vary in length, depending on the item to be labeled as well as the detection technique utilized in the nucleic acid detection process. The coding capacity of nucleic acids is for practical purposes unlimited. For instance, a ten base sequence of DNA has 1,048,576 (i.e. 4¹⁰ or over 10⁶) possible variants, so a twenty base sequence would have over 10¹² possible variants, an astronomical number of possibilities. Since modern oligonucleotide and polynucleotide synthetic machinery and methods make accessible synthetic sequences of kilobase lengths and higher, the coding capacity of these molecules is almost infinite.

In certain embodiments of the methods of the invention, the DNA marker is derived from DNA extracted from a specific plant source and is specifically digested and ligated to generate artificial nucleic acid sequences which are unique to the world. The digestion and ligation of the extracted DNA is completed by standard techniques known to those skilled in the art of molecular biology.

In certain embodiments of the methods of the invention, the nucleic acid marker is derived from DNA extracted from a specific plant source and is specifically digested and ligated to generate artificial nucleic acid sequences which are unique to the world. Once the unique sequence DNA has been produced, it can be used as a unique marker or taggant and can be included in coatings or encapsulated in other materials for protection against UV and degradation.

The marker compound can be produced as a solid or as a liquid, in water or in an oil based medium, in a suspension, in an aggregate or other suitable alternatives. One feature of the marker compounds in certain embodiments is to protect the nucleic acid fragment from UV and other factors that may degrade the DNA marker over time, while the nucleic acid is acting as an authentication tag for a particular product. In certain embodiments, DNA marker is encapsulated and suspended in a solvent solution (aqueous or organic solvent solution) producing a "stock" DNA marker solution at a specified concentration. This stock DNA solution can then easily be added to the marker compound mixture at an appropriate concentration for the type of product to be authenticated. In certain instances, the DNA marker is mixed with other components of the marker compound without any prior encapsulation. Several processes such as nucleic acid fragment encapsulation and other techniques utilized for protecting nucleotides, and in particular, DNA from degradation, are well known in the art. In one embodiment, the DNA marker can be mixed with a perturbant to aid in solubilization of DNA into and recovery of DNA from organic resins as described in US Patent application publication No. US 2014/0099643 to Jung et al.

The detection molecules of the invention can be incorporated into probe motifs, such as Taqman probes (Held et al., Genome Res. 6: 986-994 (1996), Holland et al., Proc. Nat. Acad. Sci. USA 88: 7276-7280 (1991), Lee et al., Nucleic Acids Res. 21: 3761-3766: 1993), molecular beacons; Tyagi et al., Nature Biotechnol., 16:49-53 (1998), U.S. Pat. No. 5,989,823, issued Nov. 23, 1999) scorpion probes (Whitcomb et al., Nature Biotechnology 17: 804-807: 1999), sunrise probes (Nazarenko et al., Nucleic Acids Res. 25: 2516-2521: 1997), peptide nucleic acid (PNA)-based light up probes (Kubista et al., WO 97/45539, December 1997), double-strand specific DNA dyes (Higuchi et al, Bio/Technology 10: 413-417 (1992), Wittwer et al, Bio/Techniques 22: 130-138, 1997) and the like. These and other probe motifs with which the present detection molecules can be used are reviewed in Nonisotopic DNA Probe Techniques, Academic Press, Inc. 1992.

In other embodiments, a molecular beacon system is utilized to detect and quantify the DNA marker from the product of interest. "Molecular beacons" are hairpin-shaped nucleic acid detection probes that undergo a conformational transition when they bind to their target that enables the molecular beacons to be detected. In general, the loop portion of a molecular beacon is a probe nucleic acid sequence which is complementary to the nucleic acid marker. The stem portion of the molecular beacon is formed by the annealing of arm sequences of the molecular beacon that are present on either side of the probe sequence. A functional group such as a fluorophore (e.g. coumarin, EDNAS, fluorescein, lucifer yellow, tetramethylrhodamine, texas red and the like) is covalently attached to the end of one arm and a quencher molecule such as a nonfluorescent quencher (e.g. DABCYL) is covalently attaches to the end of the other arm. When there is no target (DNA marker) present, the stem of the molecular beacon keeps the functional group quenched due to its close proximity to the quencher molecule. However, when the molecular beacon binds to their specified target, a conformational change occurs to the molecular beacon such that the stem and loop structure cannot be formed, thus increasing the distance between the functional group and the quencher which enables the presence of the target to be detected. When the functional group is a fluorophore, the binding of the molecular beacon to the DNA marker can be detected by fluorescence spectroscopy.

In other embodiments, a plurality of DNA markers with varying sequences are used in labeling a particular product. The different DNA markers can be detected quantitatively by a plurality of molecular beacons, each with a different colored fluorophore and with a unique probe sequence complementary to at least one of the plurality of DNA markers. Being able to quantitate the various fluorphores provides a higher level of authentication and security, or can be used to encode additional data. It should be noted, that the other functional groups described above useful in labeling nucleic acid probes can also be utilized in molecular beacons for the present invention.

Methods useful for incorporating DNA markers with optional optical reporters into a medium useful in the practice of the present invention, or for coating articles before exposure to the electromagnetic pulse are described in US Patent 8,426,216 to Kwok et al. Methods useful for incorporating DNA into, or coating onto articles with optical reporters and DNA into inks for secure document printing and detection useful in the practice of the present invention are described in US Patent 8,415,164. Methods useful for incorporating DNA into indicia, or coating of indicia, such as sports goods, logos or badges with optical reporters and DNA are described in US Patent 8,415,165. Methods useful for incorporating DNA into, or coating onto pharmaceutical compositions, such as tablets useful in the practice of the present invention are described in US Patent 8,420,400.

In one embodiment, the invention is useful for the marking of a wide variety of objects, such as and without limitation, electronics (including microchips, semiconductors, integrated circuits and memory chips), consumer goods, medical devices, pharmaceuticals and pharmaceutical packaging, currency, documents and IDs such as licenses and passports, metals, threads and yarns, fabrics, plastics, ceramics, automotive and aerospace parts, machine tools, jewelry and other precious objects such as gold bullion bars, and any object of interest that needs to be authenticated, tracked or traced.

In one aspect the disclosure relates to the use of LASER ablation engraving to produce a plasma at the surface of microchip that provokes the binding of DNA to the surface of microchip. In another embodiment the invention provides the use of LASER ablation engraving to produce a plasma at the surface of an integrated circuit (IC) that provokes the binding of marker DNA to the surface of the IC.

In one aspect, the authentication process includes capturing the marker DNA directly with a complementary hybridization probe attached to a solid support. In general, the methods for capturing the marker DNA involve a material in a solid-phase interacting with reagents in the liquid phase. In certain embodiments, the nucleic acid probe is attached to a solid phase. The nucleic acid probe can be in the solid phase such as immobilized on a solid support, through any one of a variety of well-known covalent linkages or non-covalent interactions. In other embodiments, the support is comprised of insoluble materials, such as controlled pore glass, a glass plate or slide, polystyrene, acrylamide gel and activated dextran. In still other embodiments, the support has a rigid or semi-rigid character, and can be any shape, e.g. spherical, as in beads, rectangular, irregular particles, gels, microspheres, or substantially flat support. In some embodiments, it may be desirable to create an array of physically separate regions on the support for sequencing with, for example, wells, raised regions, dimples, pins, trenches, rods, pins, inner or outer walls of cylinders, and the like. Other suitable support materials include, but are not limited to, agarose, polyacrylamide, polystyrene, polyacrylate, hydroxethylmethacrylate, polyamide, polyethylene, polyethyleneoxy, or copolymers and grafts of such. Other embodiments of solid-supports include small particles, non-porous surfaces, addressable arrays, vectors, plasmids, or polynucleotide-immobilizing media.

Depending on the initial concentration of the DNA marker added to the product of interest, the marker can be detected quantitatively without being amplified by PCR. In some embodiments, a single stranded DNA marker labeled with a detection molecule (i.e. fluorophore, biotin, etc.) can be hybridized to a complementary probe attached to a solid support to allow for the specific detection of the "detection molecule" configured to the DNA marker. The DNA marker can also be double stranded (dsDNA), with at least one strand being labeled with a detection molecule. With a dsDNA marker, the DNA marker must be heated sufficiently and then quick cooled to produce single stranded DNA, where at least one of the strands configured with a detection molecule is capable of hybridizing to the complementary DNA probe under appropriate hybridization conditions.

The following examples are for illustration only and should not be construed as limiting the scope of claimed the invention, which will be readily recognized by those of skill in the art.

### EXAMPLES

### EXAMPLE 1: Immobilization of DNA marker on an epoxy surface

The surface of an epoxy resin (GE8000CH4ES) is spray coated with a film of about 100 µm thickness of a DNA solution in TE buffer (10 mM Tris.HCL, 1 mM EDTA, pH 7.4). A 30 watt Firestar (Synrad, Mukilteo, WA) V30 carbon dioxide LASER beam is oriented to have 30° incidence angle relative to the epoxy surface normal. The beam focal point is arranged to be at 50 µm below the surface (spot size of 76 µm diameter) is used to create ablated materials to embed adjacent DNA coating on the edges of the beam swath. The beam progresses across the surface at a speed of 100 cm/sec using an X-Y motorized table.

### EXAMPLE 2: LASER generated plasma for immobilization of a DNA marker

A 30 watt Firestar V30 carbon dioxide LASER is used with an X-Y servomotor table at the engraving speed of 140 cm/sec and the focal point of 5 µm above the surface creating a plasma is used to activate the surface of an epoxy resin (G700HC) coated with a DNA solution in TE buffer (10 mM Tris.HCL, 1 mM EDTA, pH 7.4). The plasma produced above the DNA film is sufficient to immobilize the DNA marker on the epoxy surface.

### EXAMPLE 3: Immobilization of DNA marker on a polyimide surface

A 30 watt Quantronix Q-mark fiber LASER (Quantronics, Minneapolis, MN) operating at 80 khz is used to create an energy density of 10 joules/cm² to transfer adhesive DNA film with pigment onto the polyimide surface. The DNA marker is immobilized with a co-located visible marker pigment for identification purposes.

### EXAMPLE 4: Immobilization of DNA marker in a gold:DNA complex on an epoxy surface

A 30 watt Quantronix Q-mark fiber LASER (Quantronics, Minneapolis, MN) at an engraving speed of 50 cm/sec is used to carbonize an epoxy surface to create reactive carbon to reduce organometallic compounds. A gold:DNA complex solution is added to the LASER engraved mark for covalent bonding to the surface. The gold DNA complexes at the surface are permenantly bound.

### EXAMPLE 5: Immobilization of DNA marker in a grooved metal surface

A solid state LASER is used to engrave 600 µm wide by 200 µm depth grooves into a metal substrate surface. A 100 µm cynoacrylate fiber or metal wire encapsulating DNA and security marker is placed into the groove above. The solid state LASER is used to bond and immobilize small segments of the fiber by welding the DNA marker in the groove.

### EXAMPLE 6:

Security marker is extruded in a polyester polymer to form fibers. The fiber containing DNA is bonded to other fibers using a 30 W Firestar (Synrad, Mukilteo, WA) V30 carbon dioxide LASER with an energy density of 1 joule/cm² to form a mesh. The mesh can be used to make a fabric with DNA marker embedded therein.

### EXAMPLE 7: Encapsulation of DNA marker in waffle containers using two-photon polymerization

A Ti:Sapphire femtosecond laser (Chameleon, Coherent, Santa Clara, CA) with a wavelength of 780 nm, a pulse width <150 fs, a repetition rate of 80 MHz is used. The beam is controlled by an electrical shutter (Edmund Optics Inc., Barrington, NJ). The desired 3D object is created using Autodesk Inventor software and converted G-Code. Linux CNC software is used for positioning of the LASER focus point where the intensity is the highest for polymerization. A three-axis translation stage assembly (XMS, Newport Corp.) is used to steer the focus point to create 3D structures in dipentaerythritol pentaacrylate containing DNA. First a nanoscale open waffle tray is created with 100 nm pockets, the pockets are filled with DNA solution by spray or inkjet printing and then the LASER initiated polymerization is programmed to create a seal lid to contain DNA in each chamber. This process is optionally repeated to build up the honey comb structure one floor at a time. Excess and unpolymerized materials are removed with solvent.

### EXAMPLE 8: Encapsulation of DNA marker in segmented tubes (bamboo) using two-photon polymerization

A Ti:Sapphire femtosecond laser (High Q Laser Production GmbH) with a wavelength of 800 nm, a pulse width <100 fs, a repetition rate of 73 MHz is used. The beam is controlled by an electrical shutter (Edmund Optics Inc., Barrington, NJ). The desired 3D object is created using SolidWorks software. Labview software is used for positioning of the focus point using linear air-bearing stage (Aerotech) for creating 3D structures in Ormocer® acrylic polymer containing DNA. Four beam or three beam 100 nm diameter overlapping paths are used to create tubes for encapsulating DNA for microfabrication.

### EXAMPLE 9: Immobilization of DNA marker on 3D microfabrication using two-photon polymerization

A Ti:Sapphire femtosecond laser (MaiTai DeepSee, Spectra-Physics) with a wavelength of 775 nm, a pulse width <100 fs, a repetition rate of 80 MHz is used. The beam is controlled by an electrical shutter (Edmund Optics Inc., Barrington, NJ). The desired 3D object is created using CAD software. Labview software is used for positioning of the LASER focus point using stepper motors to create 3D structures in acrylic monomer, SR499, containing DNA. During the 3D micro fabrication or polymerization, DNA is immobilized in the polymer and on the surface. Unpolymerized monomers are removed with solvent.

### EXAMPLE 10: LASER-induced forward transfer of DNA ink

LASER-induced forward transfer (LIFT) is a versatile printing technique in which fine jets of ink are ejected from a thin donor film onto an acceptor substrate, enabling high-resolution patterns to be formed. Fluid ejections are initiated by the rapid expansion of micrometer-sized blisters that form on a polymer film underneath the layer containing the ink. Blister formation on a 7 µm film of polyimide, coated on a glass slide, is initiated by focusing a 20 ns pulse from a UV LASER (Coherent AVIA) into the film. DNA ink is ejected from the film onto the substrate. The DNA in the ink is useful for authentication of the document or object so marked.

## Claims

1. A method of marking an object, comprising:
providing an object having a surface, wherein at least a portion of the surface is coated with a detectable marker molecule;
exposing a surface of the object to be marked to a LASER pulse sufficient to immobilize the detectable marker molecule on the surface of the object; wherein the detectable marker molecule comprises a nucleic acid.

2. The method according to claim 1 , wherein the nucleic acid comprises DNA.

3. The method according to claim 1, wherein the detectable marker molecule is immobilized on at least a portion of the surface of the object by chemical bonding.

4. The method according to claim 1, wherein the detectable marker molecule is provided in a medium that is polymerized onto at least a portion of the surface of the object by the LASER pulse.

5. The method according to claim 4, wherein the detectable marker molecule is provided in a transparent photo-polymerizable medium.

6. The method according to claim 5, wherein the transparent photo-polymerizable medium comprises a two-photon polymerizable monomer.

7. The method according to claim 6, wherein the polymerization of the two-photon polymerizable monomer produces a surface coating over at least a portion of the surface of the object.

8. The method according to claim 7, wherein the surface coating includes the detectable marker molecule.

9. The method according to claim 8, wherein the detectable marker molecule is encapsulated by the surface coating.

10. The method according to claim 2, wherein the DNA is detected by hybridization, amplification or nucleotide sequencing.

11. The method according to claim 2, wherein the DNA comprises a sequence of 10 to 10,000 nucleotides.

12. The method according to claim 2, further comprising marking the object with a visible marker and/or an optical reporter.

## Patentansprüche

1. Verfahren zum Markieren eines Objekts, umfassend:
Bereitstellen eines Objekts mit einer Oberfläche, wobei mindestens ein Abschnitt der Oberfläche mit einem nachweisbaren Markermolekül beschichtet ist;
Aussetzen einer Oberfläche des zu markierenden Objekts an einen LASER-Impuls, der ausreicht, um das nachweisbare Markermolekül auf der Oberfläche des Objekts zu immobilisieren; wobei das nachweisbare Markermolekül eine Nukleinsäure umfasst.

2. Verfahren nach Anspruch 1, wobei die Nukleinsäure DNA umfasst.

3. Verfahren nach Anspruch 1, wobei das nachweisbare Markermolekül durch chemisches Binden auf mindestens einem Abschnitt der Oberfläche des Objekts immobilisiert wird.

4. Verfahren nach Anspruch 1, wobei das nachweisbare Markermolekül in einem Medium bereitgestellt ist, das durch den LASER-Impuls auf mindestens einem Abschnitt der Oberfläche des Objekts polymerisiert wird.

5. Verfahren nach Anspruch 4, wobei das nachweisbare Markermolekül in einem transparenten photopolymerisierbaren Medium bereitgestellt ist.

6. Verfahren nach Anspruch 5, wobei das transparente, photopolymerisierbare Medium ein polymerisierbares Zwei-Photonen-Monomer umfasst.

7. Verfahren nach Anspruch 6, wobei die Polymerisation des polymerisierbaren Zwei-Photonen-Monomers eine Oberflächenbeschichtung über mindestens einem Abschnitt der Oberfläche des Objekts herstellt.

8. Verfahren nach Anspruch 7, wobei die Oberflächenbeschichtung das nachweisbare Markermolekül beinhaltet.

9. Verfahren nach Anspruch 8, wobei das nachweisbare Markermolekül durch die Oberflächenbeschichtung eingekapselt ist.

10. Verfahren nach Anspruch 2, wobei die DNA durch Hybridisierung, Vergrößerung oder Nukleotidsequenzierung nachgewiesen wird.

11. Verfahren nach Anspruch 2, wobei die DNA eine Sequenz von 10 bis 10.000 Nukleotiden umfasst.

12. Verfahren nach Anspruch 2, das ferner ein Markieren des Objekts mit einem sichtbaren Marker und/oder einem optischen Reporter umfasst.

## Revendications

1. Méthode de marquage d'un objet, comprenant :
la mise en place d'un objet possédant une surface, au moins une partie de la surface étant revêtue d'une molécule marqueuse détectable ;
l'exposition d'une surface de l'objet à marquer à une impulsion LASER suffisante pour immobiliser la molécule marqueuse détectable sur la surface de l'objet ;
la molécule marqueuse détectable comprenant un acide nucléique.

2. Méthode selon la revendication 1, l'acide nucléique comprenant l'ADN.

3. Méthode selon la revendication 1, la molécule marqueuse détectable étant immobilisée sur au moins une partie de la surface de l'objet par liaison chimique.

4. Méthode selon la revendication 1, la molécule marqueuse détectable étant placée dans un milieu polymérisé sur au moins une partie de la surface de l'objet par impulsion LASER.

5. Méthode selon la revendication 4, la molécule marqueuse détectable étant placée dans un milieu photo-polymérisable transparent.

6. Méthode selon la revendication 5, le milieu photo-polymérisable transparent comprenant un monomère polymérisable à deux photons.

7. Méthode selon la revendication 6, la polymérisation du monomère polymérisable à deux photons produisant un revêtement superficiel sur au moins une partie de la surface de l'objet.

8. Méthode selon la revendication 7, le revêtement superficiel comprenant la molécule marqueuse détectable.

9. Méthode selon la revendication 8, la molécule marqueuse détectable étant encapsulée par le revêtement superficiel.

10. Méthode selon la revendication 2, l'ADN étant détecté par hybridation, amplification ou séquençage des nucléotides.

11. Méthode selon la revendication 2, l'ADN comprenant une séquence de 10 à 10 000 nucléotides.

12. Méthode selon la revendication 2, comprenant en outre le marquage de l'objet avec un marqueur visible et/ou un rapporteur optique.
